(19) Europäisches Patentamt
European Patent Office
Office européen des brevets

(11) Veröffentlichungsnummer: **0 300 160 A2**

(12) **EUROPÄISCHE PATENTANMELDUNG**

(21) Anmeldenummer: **88108282.0**

(22) Anmeldetag: **25.05.88**

(51) Int. Cl.[4]: **C07D 211/46 , C08K 5/34 , C08L 23/02**

(30) Priorität: **22.07.87 DE 3724222**

(43) Veröffentlichungstag der Anmeldung: **25.01.89 Patentblatt 89/04**

(84) Benannte Vertragsstaaten: **BE CH DE FR GB IT LI**

(71) Anmelder: **HÜLS AKTIENGESELLSCHAFT**
**Patentabteilung / PB 15 - Postfach 13 20**
**D-4370 Marl 1(DE)**

(72) Erfinder: **Disteldorf, Josef, Dr.**
**Dahlbrede 47**
**D-4370 Marl(DE)**
Erfinder: **Haage, Hans-Jürgen**
**Gaussstrasse 13**
**D-4690 Herne 2(DE)**
Erfinder: **Libera, Hubert, Dr.**
**Gaussstrasse 17**
**D-4690 Herne 2(DE)**
Erfinder: **Kirchner, Peter**
**Brünselstrasse 24**
**D-4630 Bochum 1(DE)**

(54) **Lichtstabilisatoren.**

(57) Es war die Aufgabe, Lichtstabilisatoren mit verbesserter Wirkung zu finden.

Die Lösung bestand im Auffinden von Verbindungen von der Art des gegebenenfalls noch in 1-Stellung substituierten 2,2,6,6-Tetramethyl-4-piperidinyl-docosanoats. Diese Verbindungen zeigen eine bessere Lichtstabilisierung gegenüber denen des Standes der Technik.

Stabilisierung von Polyolefinen.

EP 0 300 160 A2

## Lichtstabilisatoren

Die vorliegende Erfindung betrifft neue Lichtstabilisatoren für synthetische Polymere.

Synthetische Hochpolymere sind dafür bekannt, daß sich ihre optischen und mechanischen Eigenschaften unter dem Einfluß von Licht, Wärme oder Luftsauerstoff verschlechtern, was sich durch Risse, Kreidung, Verfärbung, Versprödung und andere unerwünschte Effekte bemerkbar macht. Zur Verbesserung der Beständigkeit werden daher den Polymeren Stabilisatoren zugesetzt, die diese Abbauerscheinungen vermindern sollen. So werden sie stets mit einem Zusatz an Antioxidantien versehen, die dem oxidativen Angriff während der Verarbeitung und der späteren Verwendung entgegenwirken. Über diese Grundstabilisierung hinaus ist es üblich, den Polymeren zur Gewährleistung einer längeren Gebrauchsdauer, insbesondere bei der Außenanwendung, Lichtstabilisatoren zuzusetzen, da die Antioxidantien die durch den UV-Anteil des Lichtes hervorgerufene Photooxidation nur wenig unterdrücken.

Ester aliphatischer, alicyclischer, aromatischer oder heterocyclischer Mono-, Di- oder Polycarbonsäuren mit 2,2,6,6-Tetramethyl-4-piperidinolen sind als Lichtstabilisatoren für synthetische Polymere allgemein bekannt und beispielsweise in den DE-OSS 19 29 928, 22 04 659, 22 58 752 und 32 33 943 beschrieben worden. Im Falle der aliphatischen Monocarbonsäureester werden dabei Ester beansprucht, die sich von Monocarbonsäuren bis zu einer C-Zahl von 18 (DE-OSS 19 29 928, 22 04 659, 32 33 943) bzw. bis zu einer C-Zahl von 21 (DE-OS 22 58 752) ableiten.

Allgemein in der Praxis durchgesetzt haben sich Polyalkylpiperidinol-ester von Dicarbonsäuren, wie Sebacinsäure oder Malonsäure, die unter dem Markenzeichen TINUVIN®770, TINUVIN®765 und TINUVIN®144 der Fa. Ciba-Geigy einen großen Marktanteil besitzen. Monocarbonsäure-ester des Polyalkylpiperidinols sind als Lichtstabilisatoren nicht erhältlich; in den veröffentlichten Vergleichsuntersuchungen (DE-OSS 19 29 928, 22 58 752) werden sie immer als den Dicarbonsäureestern unterlegen dargestellt.

Bei den zur Prüfung der Lichtschutzwirkung angewandten Bewertungskriterien, die in der Literatur beschrieben sind, handelt es sich praktisch ausschließlich um Messungen, die sich auf die mechanischen Eigenschaften eines Polymers beziehen, wie z. B. Rest-Reißfestigkeit, Rest-Dehnung, Knicktest, Carbonyl-Index etc. Lediglich bei der Stabilisierung von Lacken werden auch Kriterien wie Oberflächenglanz und Rißbildung beurteilt. Das heißt, der eigentlich bedeutendste Aspekt der Lichtschutzwirkung von sterisch gehinderten Aminen, nämlich der überragende Oberflächenschutz, wird bei der Prüfung von Polymeren ungerechtfertigterweise nicht berücksichtigt. Eine optisch einwandfreie Oberflächenbeschaffenheit ist nicht nur aus dekorativen Gründen ein wichtiges Kriterium bei der Beurteilung der Gebrauchseigenschaften eines polymeren Werkstoffes. Es hat sich in der Praxis gezeigt, daß sich bei der Kombination von sterisch gehinderten Aminen mit UV-Absorbern die Reißfestigkeit, der Knicktest usw. dann nicht mehr als aussagekräftig erweisen, wenn die Rißbildung relativ früh einsetzt. Durch die zunehmende Zerstörung der Oberfläche wird offensichtlich infolge Streuung und Absorption das Eindringen der schädigenden UV-Strahlung so weit reduziert, daß sich die mechanischen Daten längere Zeit auf höherem Niveau halten als dies bei einem System mit sehr spätem Rißbildungsbeginn der Fall ist. Trotz ausreichender mechanischer Eigenschaften ist eine Gebrauchsfähigkeit nicht mehr gegeben. Eine relativ frühe Rißbildungstendenz zeigen sterisch gehinderte Amine vom Typ der Dicarbonsäureester wie TINUVIN 770 entsprechend Bis-(2,2,6,6-tetramethyl-4-piperidinyl)-sebacat.

Aufgabe der Erfindung war es, Lichtstabilisatoren bereitzustellen, die Polymeren eine verbesserte Beständigkeit gegenüber Licht sowie thermischen und oxidativen Abbau und insbesondere einen hohen Oberflächenschutz durch geringere Rißbildung verleiht.

Obwohl also Piperidinylester allgemein bekannt sind, wurde nun gefunden, daß Ester von 2,2,6,6-Tetramethyl-4-piperidinolen mit Behensäure (Docosansäure) überraschenderweise als Lichtschutzstabilisatoren für synthetische Polymere Eigenschaften besitzen, die im Vergleich zu den bekannten Lichtschutzstabilisatoren als ungewöhnlich zu betrachten sind.

So weisen sie neben einer guten Verträglichkeit, Hydrolyse- und Extraktionsbeständigkeit eine hohe Lichtschutzwirkung sowie eine überragende Migrationsbeständigkeit auf. Die hohe Wirkung hinsichtlich der mechanischen Beständigkeit und insbesondere des Oberflächenschutzes (Rißbildung) von synthetischen Polymeren wird deutlich, wenn man gegen handelsübliche Stabilisatoren, wie z. B. TINUVIN 770, vergleicht. Darüber hinaus sorgt die gute Migrations- und Extraktionsbeständigkeit für eine lange Wirkungsdauer bei allen klimatischen Verhältnissen.

Besonders deutlich wird die Leistungsfähigkeit der Behensäureester gegenüber dem Sebacinsäurediester, wenn man äquivalente Konzentrationen an Wirkgruppen (sterisch gehinderter Piperidin-Stickstoff) beider Lichtstabilisatoren einsetzt, was beim Behensäureester aufgrund des geringeren Gehaltes praktisch eine Verdoppelung der Zugabemenge zum Polymer bedeutet. Dies ist angesichts des geringeren Preises

des Behensäureesters gegenüber dem Sebacinsäureester ohne Nachteil.

Dieses Verhalten war nicht vorhersehbar, da für die um 4 C-Atome kürzerkettige Stearinsäure (Octadecansäure) derartige vorteilhafte Eigenschaften bisher nicht bekannt sind.

Gegenstand der Erfindung sind daher Lichtstabilisatoren für synthetische Polymere, bestehend aus Estern der Behensäure (Docosansäure), gegebenenfalls im Gemisch mit geringen Anteilen an gesättigten oder ungesättigten $C_{16}$-$C_{24}$-Monocarbonsäuren, der allgemeinen Formel I

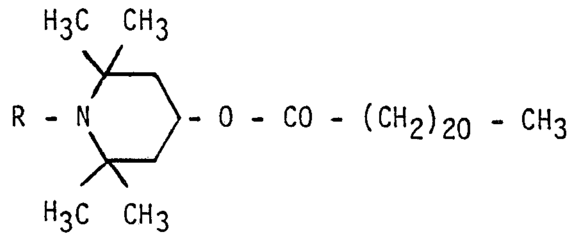

oder deren Salzen, worin R Wasserstoff, Oxylradikal, Alkyl mit 1 bis 12 C-Atomen, Alkoxialkyl mit 2 bis 21 C-Atomen, Alkenyl mit 3 bis 6 C-Atomen, Alkinyl mit 3 bis 6 C-Atomen, Benzyl oder mit Alkylresten von 1 bis 4 C-Atomen substituiertes Benzyl, 2,3-Epoxipropyl, oder ein von einer gesättigten oder ungesättigten aliphatischen Säure mit 1 bis 4 C-Atomen oder einer aliphatischen, cycloaliphatischen oder aromati schen Carbaminsäure der Formel - CO - NH - R abgeleiteten Acylrest sein kann.

Bevorzugte Beispiele für die Behensäureester sind 2,2,6,6-Tetramethyl-4-piperidinyl-docosanoat und 1,2,2,6,6-Pentamethyl-4-piperidinyl-docosanoat.

Die zur Herstellung der erfindungsgemäßen Ester eingesetzten Ausgangsstoffe 2,2,6,6-Tetramethyl-4-piperidinol und 1,2,2,6,6-Pentamethyl-4-piperidinol sind wie die Behensäure als Handelsprodukte erhältlich. Aufgrund ihres nativen Ursprungs kann die Behensäure je nach Aufarbeitungsgrad noch Beimischungen von niederen und höheren, gegebenenfalls ungesättigten Carbonsäuren sowie andere herkunftsbedingte Verunreinigungen enthalten. Zum Beispiel hat eine im Handel erhältliche Behensäure die typische Zusammensetzung von etwa 87% $C_{22}$-Säure (Behensäure), 6% $C_{20}$-Säure, 4% $C_{16}$-Säure und je 1% $C_{16}$- und $C_{24}$-Säure.

Zur Herstellung der Ester können die allgemein bekannten Verfahren der Veresterung angewendet werden:

a) Direkte Veresterung der Behensäure mit dem Polyalkyl-4-piperidinol unter Verwendung eines Katalysators, wie z. B. Aluminiumtriisopropylat oder Dibutylzinnoxid, gegebenenfalls zur Entfernung des Wassers mit einem Schleppmittel (z. B. Hexan) oder in hochsiedenden Lösemitteln (z. B. Xylol, Dekalin) mit Wasserabscheidung.

b) Umesterung eines niedrigen Alkylesters der Behensäure mit Polyalkyl-4-piperidinol in Gegenwart eines Katalysators, wie Tetraisopropyltitanat, gegebenenfalls unter Verwendung eines Lösemittes, das höher siedet als der niedrige aliphatische Alkohol des Esters.

Zur Reaktion werden die Säure-Komponente und das Polyalkyl-4-piperidinol, das als unaufgearbeitete Lösung direkt aus dem Herstellungprozeß eingesetzt werden kann, in einem Molverhältnis miteinander vermischt, daß die leichter siedende Verbindung im Überschuß vorliegt. Die Reaktion wird bei 200 °C unter Entnahme des abgespaltenen Wassers oder Alkohols durchgeführt, wonach man die nicht reagierten Anteile im Vakuum abtrennt. Die anschließende Reinigung wird durch Destillation im Dünn schichtverdampfer vorgenommen.

Die erfindungsgemäßen Behensäureester sind wirkungsvolle Stabilisatoren für organische Materialien, wie z. B. Schmieröle, Fette, Kosmetika, photographische Filme und Papiere, insbesondere aber für praktisch alle synthetischen Polymeren.

Von besonderer Bedeutung ist die Verwendung dieser Lichtstabilisatoren in Polyolefinen und Copolyolefinen, wie in Polyethylen, Polypropylen, Polyisobutylen, Poly-1-buten, Poly-1-penten, Poly-3-methyl-1-buten, Poly-4-methyl-1-penten oder in Ethylen-Propylen-Dien-Terpolymeren. Weitere separate Substrate sind ABS-Terpolymere, schlagfestes Polystyrol, Polyphenylenoxid, Polybutadien, Polyisopren, Styrol-Butadien-Kautschuk.

Die erfindungsgemäßen Lichtstabilisatoren werden den organischen Materialien in einer Menge von 0,01 bis 5 Gewichtsprozent, bezogen auf die gesamte Zusammensetzung, zugesetzt, wobei die Menge vom jeweiligen Substrat und Verwendungszweck abhängt. Vorzugsweise setzt man 0,1 bis 2,5 Gewichtsprozent zu.

Der Zusatz zu Kunststoffen kann nach üblichen Methoden in jedem beliebigen Stadium vor der

formgebenden Verarbeitung erfolgen. Beispielsweise kann der Stabilisator mit dem Polymer in Pulverform gemischt werden, oder man mischt eine Suspension oder Lösung des Stabilisators mit einer Suspension oder Lösung des Polymeren.

Die erfindungsgemäßen Lichtstabilisatoren können in Kombination mit anderen bekannten Stabilisatoren verwendet werden, wie z. B. mit Antioxidantien, Lichtschutzmittel und UV-Absorber, Metalldesaktivatoren oder Thiosynergisten.

Beispiele für solche Stabilisatoren sind z. B. Phenolderivate, Benzotriazole, Benzophenone.

Von besonderer Bedeutung ist die Mitverwendung von phenolischen Antioxidantien, wobei die verwendete Menge an Antioxidantien 0,01 bis 5 Gewichtsprozent, bezogen auf die gesamte Zusammensetzung, beträgt.

Bei der Verwendung in Kunststoffen können weitere für Kunststoffe gebräuchliche Zusätze mitverwendet werden, wie z. B. Füllstoffe, Verstärkungsmittel, Pigmente, Farbstoffe, Weichmacher, Flammschutzmittel oder Antistatika.

Die vorliegende Erfindung soll durch die nachfolgenden Beispiele weiter veranschaulicht werden.

## Beispiel 1

### 2,2,6,6-Tetramethyl-4-piperidinyl-docosanoat

In einem 500-ml-Dreihalskolben mit KPG-Rührer, Innenthermometer und kurzer Glaswendelkolonne mit Liebigkühler wurden unter einem gelinden $N_2$-Strom 102 g (0,3 mol) Behensäure (GC-Reinheit: 88,9% Docosansäure, Rest Homologe) und 70,8 g (0,45 mol) 2,2,6,6-Tetramethyl-4-piperidinol in Gegenwart von 3 g (12 mmol) Dibutylzinnoxid 24 Stunden auf 200 °C erwärmt. Man erhielt eine klare, hellbraune Reaktionslösung mit der Säurezahl 2,5 mg KOH/g (entspr. 97,5% Umsatz) und ca. 4,5 g eines wäßrigen Destillats.

Anschließend wurde das überschüssige 2,2,6,6-Tetramethyl-4-piperidinol abdestilliert, indem man den Ansatz bei Normaldruck auf 180 °C erwärmte und dann ein Vakuum von ca. 10 kPa anlegte. Das restliche Edukt wurde im Hochvakuum bei Sumpftemperaturen bis 200 °C abgezogen. Dabei wurden 21,3 g (90% des Überschusses) 2,2,6,6-Tetramethyl-4-piperidinol als heller, rückführbarer Feststoff zurückgewonnen.

Der Rückstand enthielt 136 g (entspr. 94% Ausbeute) Rohprodukt, Fp. 48 bis 53 °C, Basenzahl 116 (theor. 117 mg KOH/g), das nach Destillation im Dünnschichtverdampfer bei 210 °C/30 Pa einen farblosen Feststoff mit einem Schmelzpunkt von 50,5 bis 53 °C und einer Säurezahl <0,1 mg KOH/g ergab.

## Beispiel 2

### 2,2,6,6-Tetramethyl-4-piperidinyl-docosanoat

In einem 4-1-Vierhalskolben mit KPG-Rührer, Innenthermometer, Tropftrichter und 25-cm-Juchheimkolonne (auf 130 °C beheizt) mit Liebigkühler wurden unter einem gelinden $N_2$-Strom 102 g (0,3 mol) Behensäure (GC-Reinheit: 88,9 % Docosansäure, Rest Homologe) und 100 ml einer 18,8 %igen Lösung von 2,2,6,6-Tetramethyl-4-piperidinol in Isopropanol in Gegenwart von 3 g (4,75 mmol) Dibutylzinndilaurat auf ca. 90 °C erwärmt. Der Rest von insgesamt 375,8 g (0,45 mol) der 2,2,6,6-Tetramethyl-4-piperidinol-Lösung wurde innerhalb von 1,5 Stunden so zügig zugetropft, wie Isopropanol abdestillierte. Dann erwärmte man 24 Stunden auf 200 °C, wobei die Säurezahl des Gemisches auf den Wert von 3,4 mg KOH/g absank (d. h. 96,5 %iger Umsatz).

Anschließend wurde das nicht umgesetzte 2,2,6,6-Tetramethyl-4-piperidinol bei 180 °C/10 kPa bis 200 °C/13 Pa abdestilliert. Dabei wurden 21,7 g (92 % des Überschusses) 2,2,6,6-Tetramethyl-4-piperidinol als heller, rückführbarer Feststoff zurückgewonnen.

Das Rohprodukt (136 g, entspr. 94 % Rohausbeute) wurde im Dünnschichtverdampfer bei 210 °C/20 Pa mit ca. 150ml/h abdestilliert, wobei man neben 4,5 g dunklem Rückstand (rückführbar) 132 g (91 % Ausbeute) 2,2,6,6-Tetramethyl-4-piperidinyl-docosanoat als einen farblosen Feststoff mit einem Schmelzpunkt von 50 bis 53 °C und einer Basenzahl von 116 mg KOH/g erhielt.

Beispiel 3

1,2,2,6,6-Pentamethyl-4-piperidinyl-docosanoat

In einem 500-ml-Dreihalskolben mit KPG-Rührer, Innenthermometer und kurzer Glaswendelkolonne mit Liebigkühler wurden unter einem gelinden $N_2$-Strom 106,3 g (0,3 mol) Methyl-docosanoat und 77 g (0,45 mol) 1,2,2,6,6-Pentamethyl-4-piperidinol in Gegenwart von 2 g Aluminiumtriisopropylat 6 Stunden auf 200 °C erwärmt.

Anschließend wurde das nicht umgesetzte 1,2,2,6,6-Pentamethyl-4-piperidinol bei 10 kPa, Reste bei 20 Pa abdestilliert. Dabei wurden 24,4 g (95 % des Überschusses) 1,2,2,6,6-Pentamethyl-4-piperidinol als heller, rückführbarer Feststoff zurückgewonnen.

Der Rückstand enthielt 145,2 g (entspr. 98 % Ausbeute) Rohprodukt, das nach Destillation im Dünnschichtverdampfer mit 100 ml/h bei 220 °C/40 Pa einen farblosen Feststoff mit einem Schmelzpunkt von 53 bis 54 °C und einer Basenzahl von 117 mg KOH/g ergab.

Beispiel 4

Polypropylen mit dem Schmelzindex MFI 190/5 (DIN 53 735 Code T) von ca. 4 g/10 min und einer Dichte von 0,905 wurde in Chargen von je 800 g mit 0,1 Massenprozent Pentaerythrityl-tetrakis-3-(3,5-di-tert.-butyl-4-hydroxyphenyl)-propionat, 0,1 Massenprozent Calciumstearat und 0,25 bis 0,5 Massenprozent des zu prüfenden Lichtstabilisators in einem Labormischer (Typ Turbula, Fa. Bachofen AG, Basel) intensiv vermischt und das auf diese Weise angepuderte Granulat zwecks Homogenisierung bei einer Temperatur von 220 °C durch einen Einwellenextruder gefahren. Nach Zerkleinerung der extrudierten Polymerstränge in einer Rotoplex-Schneidmühle (Fa. Alpine) wird das Hackgranulat in einer Schneckenspritzgußmaschine (Typ: Monomat 25, Fa. Krauss-Maffei) bei 220/270/230 °C zu Platten von 60 x 60 x 1 mm verspritzt. Aus diesen wurden Probekörper für den Zugversuch (S2-Stäbe nach DIN 53 504) ausgestanzt. Die als Vergleichsmuster erforderlichen Probekörper wurden analog, jedoch ohne den zu testenden Stabilisator bzw. unter Zusatz des handelsüblichen Vergleichsstabilisators, hergestellt.

Die Ermittlung der Lichtstabilität erfolgte durch Bestrahlung der Probekörper mit Ultra-Vitalux-Lampen der Fa. Osram, die eine sonnenähnliche, von einem Quecksilberhochdruckbrenner und einer Wolframwendel erzeugte Strahlung emittieren. Die Belichtungseinrichtung war mit 16 im Quadrat angeordneten Strahlern ausgerüstet, die im Abstand von 50 cm zwischen Kolbenkuppe und Bestrahlungsgut auf einer quadratischen Fläche von 1 x 1 m eine Bestrahlungsstärke von etwa 1 $kW/m^2$ erzeugen. Am Ort der Probekörper stellte sich eine Schwarzkörpertemperatur von 55 °C ein. Während der Bestrahlung wurde weder eine Befeuchtung der Proben noch der Luft vorgenommen.

Als Maß für die Lichtschutzwirkung der zu untersuchenden Stabilisatoren diente die Expositionszeit bis zum Beginn der Bildung von Oberflächenrissen auf den bestrahlten Spitzgußplatten sowie die Halbwertszeit, unter der die Bestrahlungsdauer verstanden wird, bei der die Reißfestigkeit, nach DIN 53 455 im Zugversuch ermittelt, auf 50 % des Ausgangswertes abgefallen war.

Tabelle I zeigt die unter diesen Bedingungen erhaltenen Ergebnisse, wobei die Konzentrationsangaben in diesem und allen weiteren Beispielen in Massenprozent erfolgen, sofern nichts anderes gesagt ist.

Tabelle I

| Stabilisator | Belichtungszeit bis zum(r) Rißbildungsbeginn (h) | Halbwertszeit (h) |
|---|---|---|
| Kontrollprobe | 80 | 220 |
| 0,25 % Sebacinsäure-bis-2,2,6,6-tetramethyl-4-piperidylester | 520 | 1 090 |
| 0,25 % 2,2,6,6-Tetramethyl-4-piperidyl-docosanoat | 730 | 600 |
| 0,5 %* 2,2,6,6-Tetramethyl-4-piperidyl-docosanoat | 1 230 | 1 230 |
| 0,25 % 1,2,2,6,6-Pentamethyl-4-piperidyl-docosanoat | 750 | 690 |
| 0,5 %* 1,2,2,6,6-Pentamethyl-4-piperidyl-docosanoat | 1 200 | 1 160 |

* Diese Zusatzmenge entspricht, bezogen auf die Wirkgruppenkonzentration, dem Zusatz von 0,25 Massenprozent des handelsüblichen Vergleichsstabilisators Sebacinsäure-bis-2,2,6,6-tetramethyl-4-piperidylester.

Beispiel 5

Eine Reihe der nach dem in Beispiel 4 beschriebenen Verfahren hergestellten Prüfkörper wurden einer Ofenalterung in einem Heißluft-Trokkenschrank über 100 Stunden bei 100 °C unterworfen. Nach dieser Beanspruchung erfolgte die Bestrahlung und Untersuchung der Prüfkörper wie angegeben.

Tabelle II

| Stabilisator | Belichtungszeit bis zum Rißbildungsbeginn | |
|---|---|---|
| | ohne Ofenalterung (h) | Ofenalterung 100 h/100 °C (h) |
| 0,25 % Sebacinsäure-bis-(2,2,6,6-tetramethyl-4-piperidyl)-ester | 520 | 790 |
| 0,25 % 2,2,6,6-Tetramethyl-4-piperidyl-docosanoat | 730 | 770 |
| 0,25 % 1,2,2,6,6-Pentamethyl-4-piperidyl-docosanoat | 750 | 770 |
| 0,25 % 2,2,6,6-Tetramethyl-4-piperidyl-octadecanoat | 760 | 530 |
| 0,25 % 2,2,6,6-Tetramethyl-4-dodecanoat | 940 | 100 |
| 0,50 %* 2,2,6,6-Tetramethyl-4-piperidyl-docosanoat | 1 230 | 1 200 |
| 0,50 %* 1,2,2,6,6-Pentamethyl-4-piperidyl-docosanoat | 1 200 | 1 200 |
| 0,44 %* 2,2,6,6-Tetramethyl-4-piperidyl-octadecanoat | 1 210 | 800 |

* siehe Fußnote Tabelle I.

Beispiel 6

Eine Reihe der nach dem in Beispiel 4 beschriebenen Verfahren hergestellten Prüfkörper wurden einer extraktiven Behandlung durch 24stündige Lagerung in destilliertem Wasser von 75 °C ausgesetzt. Danach wurde wie in den vorhergehenden Beispielen belichtet und untersucht.

Tabelle III

| Stabilisator | Belichtungszeit bis zum Rißbildungsbeginn | |
|---|---|---|
| | ohne Extraktion (h) | Extraktion 24 h/75 °C (h) |
| 0,25 % Sebacinsäure-bis-(2,2,6,6-tetramethyl-4-piperidyl)-ester | 520 | 340 |
| 0,25 % 2,2,6,6-Tetramethyl-4-piperidyl-docosanoat | 730 | 510 |
| 0,25 % 1,2,2,6,6-Pentamethyl-4-piperidyl-docosanoat | 750 | 690 |
| 0,50 %* 2,2,6,6-Tetramethyl-4-piperidyl-docosanoat | 1 230 | 900 |
| 0,50 %* 1,2,2,6,6-Pentamethyl-4-piperidyl-docosanoat | 1 200 | 760 |

* siehe Fußnote Tabelle I.

## Ansprüche

1. Lichtstabilisatoren für synthetische Polymere, bestehend aus Estern der Behensäure (Docosansäure), gegebenenfalls im Gemisch mit geringen Anteilen an gesättigten oder ungesättigten $C_{16}$-$C_{24}$-Monocarbonsäuren der allgemeinen Formel I

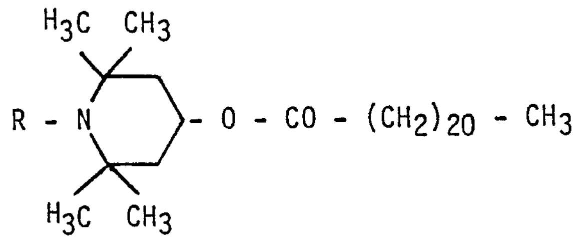

oder deren Salzen, worin R Wasserstoff, Oxylradikal, Alkyl mit 1 bis 12 C-Atomen, Alkoxialkyl mit 2 bis 21 C-Atomen, Alkenyl mit 3 bis 6 C-Atomen, Alkinyl mit 3 bis 6 C-Atomen, Benzyl oder mit Alkylresten von 1 bis 4 C-Atomen substituiertes Benzyl, 2,3-Epoxipropyl, oder ein von einer gesättigten oder ungesättigten aliphatischen Säure mit 1 bis 4 C-Atomen oder einer aliphatischen, cycloaliphatischen oder aromatischen Carbaminsäure der Formel - CO - NH - R abgeleiteten Acylrest sein kann.

2. Lichtstabilisatoren nach Anspruch 1, bestehend aus 2,2,6,6-Tetramethyl-4-piperidinyl-docosanoat.

3. Lichtstabilisatoren nach Anspruch 1, bestehend aus 1,2,2,6,6-Pentamethyl-4-piperidinyl-docosanoat.

4. Verwendung der Lichtstabilisatoren nach den Ansprüchen 1 bis 3 zur Stabilisierung von Polyolefinen, gegebenenfalls in Kombination mit an sich bekannten Stabilisatoren.